# EUROPEAN PATENT APPLICATION

(11) **EP 3 791 823 A1**
(43) Date of publication of application: **17.03.2021**
(21) Application number: 19197252.0
(22) Date of filing: 13.09.2019
(51) Int. Cl.: A61B 34/10, A61B 17/12, A61F 2/90

(54) **EXPANSION PARAMETERS OF A STENT WITH BRAIDED STRUTS**

(71) Applicant: Siemens Healthcare GmbH, 91052 Erlangen (DE)
(72) Inventor: Suzuki, Takashi, Tokyo, 142-0064 (JP)

(57) **Abstract**

The invention relates in one aspect to a method for providing expansion parameters of a stent with braided struts, an image analyzer computing unit and a computer program product.

The invention relates in one aspect to a method for providing expansion parameters of a stent with braided struts, the method comprises following steps:
- receiving a medical image in an image analyzer computing unit, wherein the medical image depicts a pathologic vessel,
- receiving an information on a stent with braided struts in the image analyzer computing unit,
- analyzing the medical image in the image analyzer computing unit, wherein the image analyzer computing unit determines expansion parameters of the stent with the braided struts depending on the pathologic vessel, wherein the expansion parameters comprise a porosity and/or a pore density of the stent with the braided struts, and
- providing the expansion parameters of the stent with the braided struts.

## Description

The invention relates in one aspect to a method for providing expansion parameters of a stent with braided struts, an image analyzer computing unit and a computer program product.

Certain types of cardiac disease and/or cerebrovascular disease, which are regularly based on a pathologic vessel, have a significant case fatality rate. One of such diseases is, for example, an aneurysm which is typically a localized bulging weak area on a blood vessel wall. A rupture of an in particular cerebral aneurysm is very dangerous for and regularly leads to death of the affected patient, if not treated within time.

Typically such aneurysm can be treated with at least three different techniques: clipping, coiling and flow diversion. The clipping technique ideally cuts off the blood supply of the aneurysm completely. The coiling technique usually comprises placing coils within the aneurysm, so blood cannot flow into the aneurysm anymore. The flow diversion technique is regularly applied, in particular if the coiling technique is not possible or favorable, and typically comprises placing a stent in the pathologic vessel with the aneurysm to divert the blood flow away from the aneurysm itself. Such stent comprises typically braided struts. Despite an increasing number of the application of the flow diversion technique, research studies are still ongoing to investigate a time frame for complete occlusion of the aneurysm and reasons for non-occluded, partially-occluded, side-branch-occluded and/or ruptured cases. The flow diversion technique can be simulated using a virtual stent technique on basis of a vessel morphology and/or a stent specification and/or a computer-aided design model of such stent.

US 2019/0038358 A1 discloses a method of estimating a length of stent.

The underlying technical problem of the invention is to disclose a method for providing expansion parameters of a stent with braided struts, an image analyzer computing unit and a computer program product for providing a real stent configuration.

This problem is solved by the features of the independent claims. Advantageous embodiments are disclosed within the dependent claims.

The invention relates in one aspect to a method for providing expansion parameters of a stent with braided struts, the method comprises following steps:
- receiving a medical image in an image analyzer computing unit, wherein the medical image depicts a pathologic vessel,
- receiving an information on a stent with braided struts in the image analyzer computing unit,
- analyzing the medical image in the image analyzer computing unit, wherein the image analyzer computing unit determines expansion parameters of the stent with the braided struts depending on the pathologic vessel, wherein the expansion parameters comprise a porosity and/or a pore density of the stent with the braided struts, and
- providing the expansion parameters of the stent with the braided struts.

The invention is in particular advantageous since it can provide to a user real expansion parameters with respect to the pathologic vessel and typically thereby a real stent configuration. Advantageously, the expansion parameters can be provided automatically, e.g. in a colored visualization of the pathologic vessel and/or of the stent with the braided struts. Another advantage is that the analyzed medical image, in particular the provided expansion parameters, can be input for a simulation, for example a computational fluid dynamics analysis. The expansion parameters allow advantageously an assessment of a hemodynamic performance of the stent with the braided struts. The provided expansion parameters are in particular advantageous, since a reduction effect on a blood flow through the stent with the braided struts depends typically on the expansion parameters. The reduction effect is typically the larger, the lower the porosity is and/or the higher the pore density is. Another advantage of the invention can be that the provided expansion parameters allow for research on and/or avoidance of aneurysm occlusion in response on the stent with the braided struts and/or for predicting of a prognosis in response on the stent with the braided struts. Advantageously, the provided expansion parameters allow for assessing whether the pathologic vessel, in particular the aneurysm, will be occluded before rupture and/or whether a perforator vessel close to the pathologic vessel, in particular the aneurysm, is excluded from occlusion.

The method for providing the expansion parameters of the stent with the braided struts is typically a computer-implemented method. The method for providing the expansion parameters of the stent with the braided struts is advantageously a method for providing the expansion parameters of the stent with the braided struts for endovascular treatment of a cerebral aneurysm of a patient. The cerebral aneurysm can be part of the pathologic vessel.

Typically, a patient comprises the pathologic vessel. The pathologic vessel can be part of a pathologic vascular tree. The pathologic vessel can be a cardiac vessel or a cerebrovascular vessel or another vessel. Usually a part of the pathologic vessel comprises a pathology. The pathology can comprise in particular an aneurysm and/or a vessel stenosis and/or an occluded vessel and/or another malfunction of a vessel.

The initial form of the stent with the braided struts is typically cylindrical, in particular with a hollow diameter. The braided struts form usually the lateral area of the axial open and cylindrical stent. The braided struts are typically helically braided. A first number of struts is typically radiopaque and a second number of struts is typically radiotransparent. The second number is regularly higher than the first number. The radiopaque struts can regularly be visualized using X-rays and comprise typically metal. Usually the radiotransparent struts cause no attenuation of the X-rays and therefore cannot and/or can hardly be visualized and are usually metal-free. Braided struts can look like to be crossed. Some of the braided struts typically look like to be crossed with an angle smaller than 180°. The struts are typically wires. At least one of the struts is ideally rotationally symmetric such as a single stretched wire. The struts can comprise a single diameter or different diameters. A diameter of the stent with the braided struts is typically smaller than 50 mm, advantageously smaller than 10 mm, in particular advantageously smaller than 5 mm. The diameter of the stent with the braided struts and/or the crossing angle of the braided struts can typically be varied by expansion. When the stent with the braided struts is navigated typically using a catheter throughout the vascular tree of the patient, the diameter of the stent with the braided struts is typically smaller than the diameter of the stent with the braided struts expanded at the pathologic vessel.

The stent with the braided struts can be at least one of the following flow diverter stent, coil-assist stent and/or stent for carotid artery stenosis. The stent with the braided struts advantageously diverts the blood flow entering the pathology of the pathologic vessel, in particular entering the aneurysm. The stent with the braided struts usually limits and/or cuts off the blood flow into the pathology of the pathologic vessel.

The porosity is typically defined as a proportion of the strut-free lateral area to the total lateral area, in particular given in [%]. The porosity depends typically on the pathologic vessel and/or is usually between 20 and 90%, advantageously between 60 and 80%. The strut-free lateral area is typically formed by several pores between the braided struts. A pore between the braided struts is typically rhombic. The pore density is typically defined as a number of pores per lateral area, in particular given in [# of pores/mm^2]. The pore density depends usually on the pathologic vessel and/or is usually between 1 and 200 pores/mm^2, advantageously between 10 and 70 pores/mm^2. The pore density correlates typically with the number of struts.

The expansion parameters are typically spatially resolved determined. The expansion parameters are in particular expansion parameters adapted to the pathologic vessel. The expansion parameters can vary based on a location within the stent with the braided struts. In one embodiment, the image analyzer computing unit determines the expansion parameters of the stent with the braided struts depending on a curvature of the pathologic vessel. A curvature of the stent with the braided struts typically depends on the curvature of the pathologic vessel. In particular the braided struts of the stent reflect the curvature of the pathologic vessel, especially if the stent is expanded, and therefore the determined expansion parameters typically depend on the curved struts. Advantageously, the expansion parameters reflect the curvature of the pathologic vessel and/or of the stent with the braided struts.

The medical image is for example acquired in a magnetic resonance imaging device, in a computed tomography device, in a C-arm angiography device and/or in a conventional x-ray imaging device. The medical image can typically be stored in a DICOM image format. The image analyzer computing unit comprises in particular an interface for receiving the medical image. The medical image can be a single medical image or alternatively a time-resolved and/or three-dimensional medical image series. The medical image usually comprises the pathologic vessel or at least an image of the pathologic vessel. The medical image is typically acquired by imaging the pathologic vessel before the medical image is received in the image analyzer computing unit. The medical image can be received from a picture archiving system.

The information on the stent with the braided struts can be an image information, a text information, an encoded information, an audio information or a combination of the previous. In particular, the information on the stent with the braided struts comprises at least one parameter of the following:
- a type of the stent with the braided struts,
- an expansion characteristic of the stent with the braided struts,
- a material of the stent with the braided struts. The type of the stent with the braided struts can be an identifier of the stent with the braided struts, in particular provided by a manufacturer of the stent with the braided struts. Alternatively, the image analyzer computing unit, in particular if the type of the stent with the braided struts is given at least partially as image information, could determine and/or recognize the type of the stent with the braided struts, for example, using a convolutional neural network and/or an atlas. The expansion characteristic is typically dependent on the pathologic vessel. The expansion characteristic describes advantageously an expansion behavior of the stent with the braided struts. A difference between the expansion characteristic and the expansion parameters is that the expansion parameters are usually determined after expansion of the stent with the braided struts whereas the expansion characteristic parameterizes typically the expansion process itself. For example, the expansion characteristic is independent on the expansion of the stent with the braided struts. Usually the expansion parameters are dependent on the expansion of the stent with the braided struts. The expansion characteristic can depend on the material of the stent with the braided struts. The material of the stent with the braided struts and/or in particular of the radiopaque struts comprises typically one of the following Pt, W, Cr, Co and/or an alloy thereof.

The image analyzer computing unit is typically configured to determine the expansion parameters of the stent with the braided struts. If the medical image and the information on the stent with the braided struts is provided as input for the image analyzer computing unit, the image analyzer computing unit can typically determine the expansion parameters as output of the analyzing. The image analyzer computing unit can comprise another interface or use the interface for receiving the information on the stent with the braided struts. In one advantageous embodiment the image analyzer computing unit determines both expansion parameters, the porosity and the pore density of the stent with the braided struts.

The analyzing can comprise an image segmentation and/or an image extraction and/or an image filter algorithm of the medical image. Alternatively or additionally, the determining can comprise applying a trained function and/or a machine learning unit on the medical image and/or the information on the stent. The trained function can be represented in the convolutional neural network or in another artificial neural network. The convolutional neural network is typically trained with training data to determine the expansion parameters of the stent with the braided struts depending on the pathologic vessel.

The providing the expansion parameters of the stent with the braided struts can comprise, in particular color-coded, visualizing at least one of the expansion parameters on a display unit. Additionally or alternatively, the providing the expansion parameters of the stent with the braided struts can comprise storing the expansion parameters in the picture archiving system. The providing the expansion parameters can comprise storing the expansion parameter for further processing in particular by the user or the image analyzer computing unit. The providing the expansion parameters can mark the end of determining the expansion parameters.

One embodiment of the invention relates in one aspect to that the information on the stent with the braided struts is selected from a plurality of stents with braided struts and that the stent with the braided struts is selected to be placed in the pathologic vessel in dependence on the expansion parameters during a subsequent treatment procedure. This embodiment is in particular advantageous since the expansion parameters are determined before the stent with the braided struts is placed in the pathologic vessel. The placement of the stent with the braided struts typically comprises the expansion of the stent with the braided struts in the pathologic vessel. Another advantage of this embodiment is that a user of the image analyzer computing unit is supported at selecting the stent with the braided struts. The outcome of the placement of the selected stent can typically be evaluated before the placement takes place during the subsequent treatment procedure. The treatment procedure can be an invasive and/or can include to place the stent with the braided struts in the pathologic vessel. The subsequent treatment procedure is typically not part of the subject-matter of this embodiment. In particular advantageous is if the image analyzer computing unit is configured to select the stent with the braided struts to be placed in the pathologic vessel usually after having selected the information on the stent with the braided struts from the plurality of stents with braided struts. The image analyzer computing unit is in particular configured to recommend a stent with the braided struts to be placed in the pathologic vessel to the user in dependence on the expansion parameters. The expansion parameters according to this embodiment are in particular prospectively determined. In this particular embodiment the information on the stent can in particular comprises the expansion characteristic. Typically the image analyzer computing unit is configured to simulate an expansion of at least one stent of the plurality of stents with braided struts in dependence on the medical image, in particular on the pathologic vessel. The simulation can comprise an image registration and/or image segmentation algorithm. The selection of the stent with the braided struts to be placed in the pathologic vessel can in particular comprise to apply another trained function, wherein the trained function can be represented in the convolutional neural network or in the another artificial neural network or in a third neural network.

Another aspect of the previous embodiment relates to that at least one threshold expansion parameter of the stent with the braided struts is received in the image analyzer computing unit, wherein the at least one threshold expansion parameter with at least one of the determined expansion parameters are compared with each other and wherein in dependence on the result of the comparison the stent with the braided struts is exchanged. This embodiment advantageously defines the selection of the stent with the braided struts to the placed in the pathologic vessel. The at least one threshold expansion parameter can relate to an outcome of previous treatment procedures and/or to recommended expansion parameters of a medical textbook. The at least one threshold expansion parameter can relate to a probability of a specific outcome. This embodiment is in particular advantageous since the image analyzer computing unit can support the user in a decision-making process which stent with the braided struts is to be placed in the pathologic vessel for the best possible outcome.

One alternative embodiment of the invention relates in one aspect to that the information on the stent with the braided struts is included in the medical image in addition to the pathologic vessel at acquiring the medical image and that the image analyzer computing unit determines the expansion parameters depending on the acquired pathologic vessel and the acquired stent with the braided struts. Usually, the stent with the braided struts is placed in the pathologic vessel before the information on the stent with the braided struts is received in the image analyzer computing unit. The expansion parameters according to this embodiment are in particular retrospectively determined. Typically, in this embodiment the received information on the stent with the braided struts is the image information. The image information is in particular included in the medical image. The medical image in particular comprises the information on the stent with the braided struts and the pathologic vessel. The acquiring the medical image with the information on the stent and with the pathologic vessel typically occurs before receiving the information on the stent. Typically, the analyzing the medical image in the image analyzer computing unit comprises a modelling of the acquired stent with the braided struts prior to determining the expansion parameters, for example to derive a configuration and/or shape of the stent with the braided struts. The modelling of the acquired stent can result in determining the type of the stent with the braided struts. Additionally or alternatively, the modelling of the acquired stent with the braided struts comprises interpolating the acquired stent with the braided struts. For example, the medical image, in particular the received image information on the stent with the braided struts, comprises the radiopaque struts. Since a signal to noise ratio of the radiopaque struts is typically higher than a signal to noise ratio of the radiotransparent struts, the radiotransparent struts are calculated by said interpolation based on the radiopaque struts. The expansion parameters are determined typically dependent on the acquired radiopaque struts and on the interpolated radiotransparent struts. This embodiment is in particular advantageous since a user, for example having placed the stent with the braided struts in the pathologic vessel, can assess the provided real expansion parameters of the stent with the braided struts depending on the pathologic vessel.

Another aspect of the previous embodiment relates to that the at least one threshold expansion parameter of the stent with the braided struts is received in the image analyzer computing unit and that the at least one threshold expansion parameter with at least one of the determined expansion parameters are compared with each other and wherein in dependence on the result of the comparison a position of the stent with the braided strut is changed during another subsequent treatment procedure. Ideally the image analyzer computing unit is configured to recommend the change or an improved position of the stent with the braided struts. This embodiment is in particular advantageous since the image analyzer computing unit supports ideally the user in a decision-making process how to place the stent with the braided struts in the pathologic vessel for the best possible outcome.

The image analyzer computing unit comprises a data processing unit and a memory unit. The data processing unit can be realized as a data processing system or as a part of a data processing system. The data processing system can, for example, comprise cloud-computing system, a computer network, a computer, a tablet computer, a smartphone or the like. The data processing system can comprise hardware and/or software. The hardware can be, for example, a processor, a memory and combinations thereof. The hardware can be configurable by the software and/or be operable by the software.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

Reference is made to the fact that the described methods and the described data processing unit as well as the described imaging device are merely preferred example embodiments of the invention and that the invention can be varied by a person skilled in the art, without departing from the scope of the invention provided it is specified by the claims. Said features, advantages or alternative embodiments of the apparatus apply also to the method and vice-versa.

The invention will be illustrated below with reference to the accompanying figures using example embodiments. The illustration in the figures is schematic and highly simplified and not necessarily to scale.
Fig. 1 shows a diagram illustrating a method for providing expansion parameters of a stent with braided struts according to one aspect of the invention,
Fig. 2 shows a diagram illustrating the method for providing the expansion parameters of the stent with the braided struts according to another aspect of the invention,
Fig. 3 shows a diagram illustrating the method for providing the expansion parameters of the stent with the braided struts according to a third aspect of the invention and
Fig. 4 and Fig. 5 show exemplary illustrations of different expansion parameters.

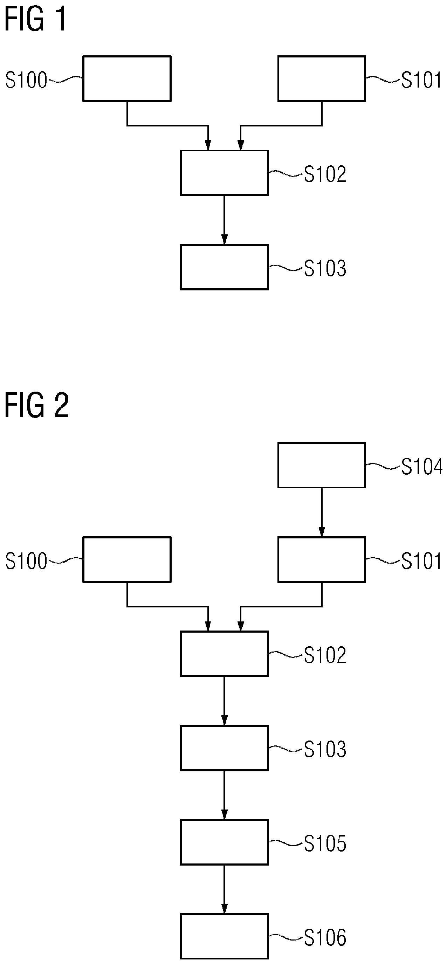

**Fig. 1** shows a diagram illustrating a method for providing expansion parameters of a stent with braided struts according to one aspect of the invention.

Method step S100 includes receiving a medical image in an image analyzer computing unit, wherein the medical image depicts a pathologic vessel.

Method step S101 includes receiving an information on a stent with braided struts in the image analyzer computing unit. The stent with the braided struts can be at least one of the following flow diverter stent, coil-assist stent and/or stent for carotid artery stenosis. The information on the stent with the braided struts comprises typically at least one parameter of the following:
- a type of the stent with the braided struts,
- an expansion characteristic of the stent with the braid-ed struts,
- a material of the stent with the braided struts.

Method step S102 includes analyzing the medical image in the image analyzer computing unit, wherein the image analyzer computing unit determines expansion parameters of the stent with the braided struts depending on the pathologic vessel, wherein the expansion parameters comprise a porosity and/or a pore density of the stent with the braided struts. The image analyzer computing unit determines usually the expansion parameters of the stent with the braided struts depending on a curvature of the pathologic vessel.

Method step S103 includes providing the expansion parameters of the stent with the braided struts.

**Fig. 2** shows a diagram illustrating the method for providing the expansion parameters of the stent with the braided struts according to another aspect of the invention on basis of the diagram of Fig. 1.

Method step S104 includes that the information on the stent with the braided struts is selected from a plurality of stents with braided struts.

Method step S105 includes that at least one threshold expansion parameter of the stent with the braided struts is received in the image analyzer computing unit, wherein the at least one threshold expansion parameter with at least one of the determined expansion parameters are compared with each other and wherein in dependence on the result of the comparison the stent with the braided struts is exchanged.

Method step S106 includes that the stent with the braided struts is selected to be placed in the pathologic vessel in dependence on the expansion parameters during a subsequent treatment procedure.

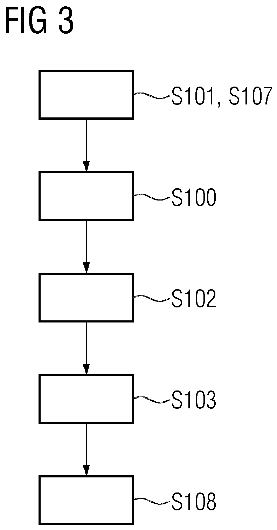

**Fig. 3** shows a diagram illustrating the method for providing the expansion parameters of the stent with the braided struts according to a third aspect of the invention on basis of the diagram of Fig. 1. Fig. 3 shows an alternative embodiment to the embodiment shown in Fig. 2.

Method step S107 includes that the information on the stent with the braided struts is included in the medical image in addition to the pathologic vessel at acquiring the medical image and that the image analyzer computing unit determines the expansion parameters depending on the acquired pathologic vessel and the acquired stent with the braided struts.

Method step S108 includes that at least one threshold expansion parameter of the stent with the braided struts is received in the image analyzer computing unit and that the at least one threshold expansion parameter with at least one of the determined expansion parameters are compared with each other and that in dependence on the result of the comparison a position of the stent with the braided strut is changed during another subsequent treatment procedure.

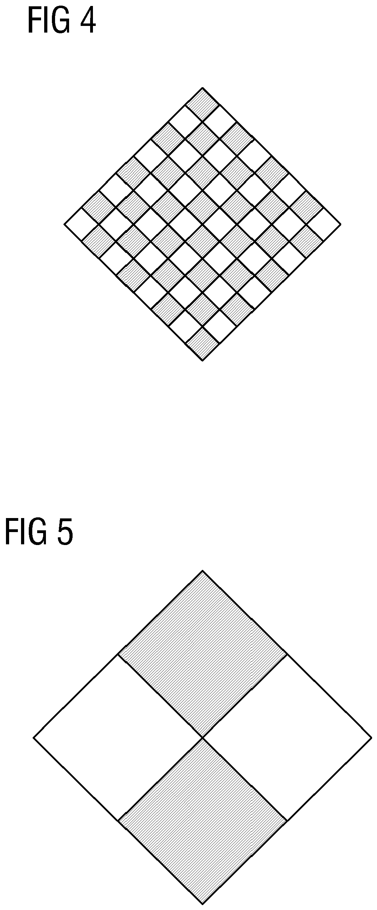

**Fig. 4** illustrates exemplary expansion parameters with porosity = 50% and pore density = 128 pores/mm^2. The black pixel representing the struts (irrespective whether radiopaque or radiotransparent) and the white pixel representing the rhombic pores. The main edge length equals 1 mm.

**Fig. 5** illustrates exemplary expansion parameters with porosity = 50% and pore density = 2 pores/mm^2.

## Claims

1. A method for providing expansion parameters of a stent with braided struts, the method comprising:
- receiving a medical image in an image analyzer computing unit, wherein the medical image depicts a pathologic vessel,
- receiving an information on a stent with braided struts in the image analyzer computing unit,
- analyzing the medical image in the image analyzer computing unit, wherein the image analyzer computing unit determines expansion parameters of the stent with the braided struts depending on the pathologic vessel, wherein the expansion parameters comprise a porosity and/or a pore density of the stent with the braided struts, and
- providing the expansion parameters of the stent with the braided struts.

2. The method of claim 1, wherein the image analyzer computing unit determines the expansion parameters of the stent with the braided struts depending on a curvature of the pathologic vessel.

3. The method of any one of the preceding claims, wherein the stent with the braided struts is at least one of the following flow diverter stent, coil-assist stent and/or stent for carotid artery stenosis.

4. The method of any one of the preceding claims, wherein the information on the stent with the braided struts comprises at least one parameter of the following:
- a type of the stent with the braided struts,
- an expansion characteristic of the stent with the braided struts,
- a material of the stent with the braided struts.

5. The method of any one of the preceding claims, wherein the information on the stent with the braided struts is selected from a plurality of stents with braided struts and wherein the stent with the braided struts is selected to be placed in the pathologic vessel in dependence on the expansion parameters during a subsequent treatment procedure.

6. The method of claim 5, wherein at least one threshold expansion parameter of the stent with the braided struts is received in the image analyzer computing unit, wherein the at least one threshold expansion parameter with at least one of the determined expansion parameters are compared with each other and wherein in dependence on the result of the comparison the stent with the braided struts is exchanged.

7. The method of any one of the claims 1 to 4, wherein the information on the stent with the braided struts is included in the medical image in addition to the pathologic vessel at acquiring the medical image and wherein the image analyzer computing unit determines the expansion parameters depending on the acquired pathologic vessel and the acquired stent with the braided struts.

8. The method of claim 7, wherein at least one threshold expansion parameter of the stent with the braided struts is received in the image analyzer computing unit and wherein the at least one threshold expansion parameter with at least one of the determined expansion parameters are compared with each other and wherein in dependence on the result of the comparison a position of the stent with the braided strut is changed during another subsequent treatment procedure.

9. Image analyzer computing unit, comprising
- a data processing unit and
- a memory unit, wherein the image analyzer computing unit is configured to any one of the preceding claims.

10. A computer program product comprising a computer program, the computer program being loadable into a memory unit of a processor, including program code sections to make the processor execute the method of any one of claims 1 to 8 when the computer program is executed in said processor.
